# FASCICULE DE BREVET EUROPEEN

(11) **EP 2 268 208 B1**
(45) Date de publication et mention de la délivrance du brevet: **08.06.2016**
(21) Numéro de dépôt: 09726876.7
(22) Date de dépôt: 27.02.2009
(51) Int. Cl.: A61B 8/08, A61B 8/12, A61B 1/00

(54) **DISPOSITIF DE MICRO-ELASTOGRAPHIE**
VORRICHTUNG FÜR MIKRO-ELASTOGRAPHIE
DEVICE FOR MICRO-ELASTOGRAPHY

(30) Priorité: 29.02.2008 FR 0851345
(43) Date de publication de la demande: 05.01.2011
(73) Titulaire: Echosens, 75013 Paris (FR)
(72) Inventeur: SANDRIN, Laurent, F-92240 l'Hay-les-Roses (FR)
(74) Mandataire: Lebkiri, Alexandre
(86) Numéro de dépôt international: PCT/FR2009/000218
(87) Numéro de publication internationale: WO 2009/122024

(56) Documents cités:
- EP-A- 0 920 833
- WO-A-2004/016176
- US-A- 5 588 432
- US-A- 5 993 389
- US-A1- 2002 068 870
- US-A1- 2005 203 398

## Description

La présente invention concerne un dispositif et un procédé pour mesurer des propriétés viscoélastiques, dénommées PV par la suite, d'un milieu viscoélastique et plus particulièrement d'un tissu biologique humain ou animal.

On connaît les documents US5993389, US5588432 et EP0920833 qui divulguent des procédés permettant de visualiser des tissus au moyen d'ultrasons.

La présente invention se rapporte plus particulièrement à un procédé et à un dispositif de micro-élastographie, dénommé DME par la suite, de mesures quantitatives et qualitatives de propriétés viscoélastiques telles que l'élasticité et/ou la viscosité d'un tissu biologique humain ou animal mis en oeuvre à l'intérieur du corps humain ou animal.

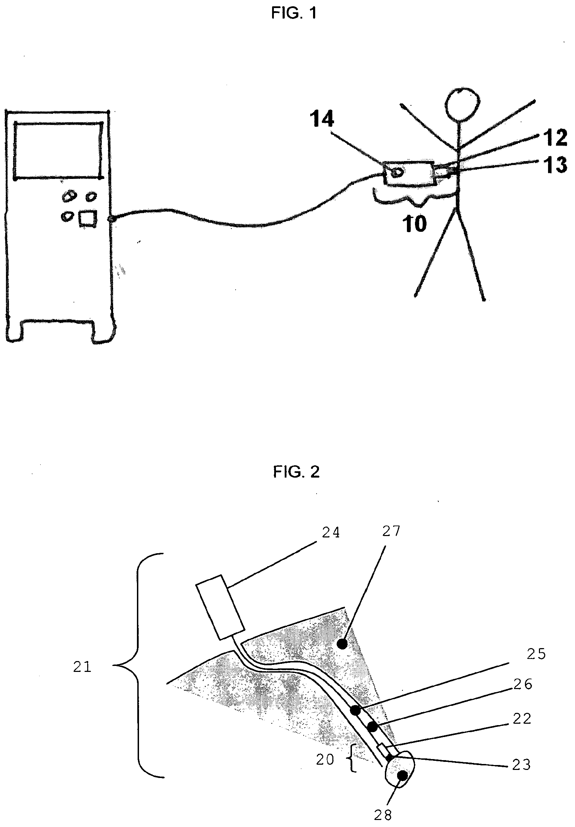

Afin de mesurer les PV d'un tissu biologique, il est connu d'utiliser l'élastométrie impulsionnelle comme décrit, par exemple, dans la demande de brevet FR 2843290 déposée le 8 août 2002 au nom de la société ECHOSENS Société Anonyme. La mise en oeuvre d'un tel procédé s'effectue à l'aide d'une sonde 10 (figure 1) munie d'un générateur de vibration 12 générant une onde élastique basse fréquence dans un tissu, par exemple par vibration d'un palpeur, et analysant la propagation de cette onde basse fréquence à l'aide d'ondes ultrasonores émises et reçues par un transducteur ultrasonore 13 pendant la propagation de l'onde élastique basse fréquence. Il est important de noter que dans cette mise en oeuvre le transducteur ultrasonore vibre au contact des tissus. La sonde 10 munie du générateur de vibration 12 et du transducteur ultrasonore 13 présente également un contrôleur 14 commandant ces derniers. Ce procédé permet de mesurer les PV d'un organe situé à proximité de l'épiderme contre lequel est mise en contact la sonde 10.

Un procédé et un tel dispositif présentent des inconvénients. Notamment, ils ne permettent pas la mesure des tissus appartenant à des organes situés en profondeur dans le corps humain. En effet, la propagation de ou de(s) onde(s) élastique(s) basse fréquence dans un corps est d'autant plus perturbée par l'hétérogénéité de ce dernier que cette ou ces ondes progressent profondément dans le corps.

En outre, lorsque l'onde basse fréquence est générée, un phénomène de diffraction proche de sa source - le générateur de vibration - se produit sur une épaisseur qui dépend entre autre des dimensions de cette source, des PV du milieu et de la fréquence de l'onde élastique basse fréquence.

Cette épaisseur minimum, en deçà de laquelle la mesure est impossible, est de l'ordre de 10 mm pour une fréquence centrale de l'onde élastique basse fréquence de 50 Hz. Pour limiter ce problème, il est fait abstraction des données sur cette épaisseur dans le calcul des PV.

En outre, un tel procédé est confronté au problème des tissus adipeux intercalés entre l'épiderme et le tissu à mesurer lorsqu'il est appliqué à un corps. En effet, les tissus adipeux déforment et atténuent les ondes ultrasonores haute fréquence et élastiques basse fréquence ce qui rend difficile leur observation au-delà d'une profondeur maximum d'observation. Ainsi, une couche adipeuse d'une épaisseur supérieure à 25 mm empêche la mesure des PV des tissus situés derrière cette couche adipeuse.

Un inconvénient supplémentaire réside dans la nécessité pour le praticien de maintenir manuellement un contact satisfaisant entre l'épiderme et le dispositif, et notamment son transducteur ultrasonore. De fait, pour obtenir une propagation optimale, l'extrémité du transducteur ultrasonore doit se trouver perpendiculaire au tissu dont on souhaite mesurer les PV et les variations du contact peuvent altérer la mise en oeuvre du procédé.

Afin de remédier à au moins un des inconvénients mentionnés ci-dessus, la présente invention, qui est définie dans les revendications, concerne un dispositif d'élastographie vibratoire pour la mesure quantitative et/ou qualitative de propriétés viscoélastiques d'un tissu humain ou animal, ce dispositif étant muni:
- d'une sonde comprenant au moins un transducteur ultrasonore et un générateur de vibration basse fréquence, le(s) transducteur(s) ultrasonore générant des ondes ultrasonores permettant l'analyse de la propagation des ondes élastiques basse fréquence se propageant dans l'organe et générées par le générateur de vibration basse fréquence, la sonde étant destinée à être positionnée au voisinage ou au contact de l'organe,
- d'un contrôleur, relié à la sonde, comprenant des moyens d'actionnement de la sonde, le contrôleur étant destiné à être maintenu à l'extérieur du corps humain ou animal,
- des moyens de liaison mécanique de la sonde avec le contrôleur, les moyens de liaison mécanique étant formés par un tube filiforme.

On entend notamment par tube filiforme un tube, une gaine, un tuyau ou une canule allongé et mince c'est-à-dire de faible diamètre ou de faible épaisseur, ce tube filiforme pouvant être souple, flexible ou rigide.

Un tel dispositif, dénommé DME pour Dispositif de Micro Elastographie, permet de mesurer quantitativement et/ou qualitativement les PV d'un tissu humain ou animal profond, c'est-à-dire interne au corps humain ou animal, en amenant la sonde munie d'au moins un transducteur ultrasonore et du générateur de vibration au voisinage ou au contact de ce tissu profond permettant ainsi de s'affranchir de l'hétérogénéité du corps humain ou animal et également de l'épaisseur de la couche adipeuse.

De préférence ledit tube filiforme présente une longueur supérieure à 20 mm, de préférence comprise entre 20 mm et 3 mètres.

Avantageusement le tube filiforme reliant la sonde interne au générateur de vibration externe est flexible, libre de contrainte angulaire ou rigide.

En outre selon une possibilité avantageuse offerte par l'invention le tube filiforme est le générateur de vibration.

Avantageusement le transducteur ultrasonore présente un diamètre actif, correspondant au diamètre d'émission et d'acquisition ultrasonore, inférieur à 3 mm.

Selon un mode de réalisation préféré de l'invention, ledit dispositif comprend un tube filiforme formé par un cathéter, une aiguille ou un endoscope dont l'extrémité distale contient la sonde et l'extrémité proximale comprend le contrôleur.

Par ailleurs suivant le champ d'investigation les endoscopes peuvent être flexible ou rigide et sont nommés en conséquence : bronchoscopes, gastroscopes, duodénuoscope, rectoscopes, laparoscopes, arthroscopes etc.
Parallèlement les aiguilles peuvent être flexibles ou rigides, de biopsie, radiofréquence, etc.

Selon un mode de réalisation préféré de l'invention ledit tube filiforme forme un fourreau dans lequel une aiguille est introduite.

Avantageusement ledit contrôleur comprend des moyens pour commander la transmission d'énergie au générateur de vibration et/ou au(x) transducteur(s) ultrasonore.

La présente invention porte également sur un procédé d'élastographie vibratoire pour la mesure quantitative et/ou qualitative de propriétés viscoélastiques d'un tissu humain ou animal mettant en oeuvre un dispositif selon l'une quelconque des revendications 1 à 12 consistant à :
- positionner la sonde au voisinage ou au contact du tissu à mesurer,
- maintenir au contact du tissu, au moins un transducteur ultrasonore pendant la réalisation de l'étape d'émission et d'acquisition ultrasonore et de génération d'une ou de plusieurs onde(s) élastique(s) basse fréquence,
- générer une ou des onde(s) élastique(s) basse fréquence,
- simultanément à l'étape précédente, générer des émissions ultrasonores et acquérir des signaux ultrasonores haute fréquence à cadence élevée pendant la propagation de de l'onde ou des ondes élastiques basse fréquence,
- calculer les variations spatio-temporelles des déplacements et/ou des déformations et/ou des vitesses de déplacement et/ou des vitesses de déformations engendrés dans l'organe ou plus généralement de tout paramètre de mouvement,
- calculer les propriétés viscoélastiques du tissu.

A cet effet, la sonde et une partie du tube filiforme sont introduit par des voies, naturelles (voies aériennes, bouche, nez, rectum....) par voie percutanée c'est-à-dire au travers de la peau, par des voies artificielle (canule, écarteur chirurgical, trocart, ...), ou par des voies matérielles (canal opérateur d'un endoscope, cathéter, ...) afin d'être acheminée au voisinage ou au contact direct avec le tissu dont les PV sont déterminées.

Avantageusement, ledit tube filiforme introduit dans le canal opérateur d'un endoscope est orienté au moyen d'un érecteur équipant un endoscope afin de positionner l'extrémité distale du dispositif en regard des tissus à mesurer.

Avantageusement ladite sonde et une partie du tube filiforme sont introduites dans un liquide appartenant au corps humain ou animal.

De préférence l'émission ultrasonore haute fréquence est réalisée sur une gamme de fréquence comprise entre 1 MHz et 200 MHz, et plus spécifiquement entre 5 MHz et 50 MHz.

Avantageusement lesdites ondes élastiques basse fréquence sont générées sur une gamme de fréquence comprise entre 5 Hz et 2000 Hz.

Selon une mise en oeuvre préférée de l'invention des ondes élastiques basse fréquence sont générées par vibration mécanique, par pression de radiation, par hyperthermie ou par vibration naturelle des tissus out tout autre type d'énergie capable de générer une ou de(s) vibration(s) basse fréquence.

D'autres caractéristiques et avantages de l'invention apparaîtront à la lumière de la description effectuée ci-dessous, à titre illustratif et non limitatif, de modes de réalisations faisant références aux figures ci-jointes sur lesquelles :
- la figure 1 illustre schématiquement un dispositif d'élastographie conforme à l'art antérieur,
- la figure 2 représente schématiquement la mise en oeuvre d'un DEM dont le tube filiforme forme un cathéter ou un endoscope,
- la figure 3 illustre un DME conforme à l'invention mis en oeuvre au sein du tractus gastro-intestinal humain pour la mesure de PV relatives au pancréas à l'aide d'un cathéter ou d'un endoscope,
- la figure 4 représente une mise en oeuvre d'un DME conforme à l'invention dans le tractus gastro-intestinal humain pour la mesure de PV relatives à l'estomac ou au foie à l'aide d'un endoscope,
- la figure 5 est une vue de détail d'une sonde d'un DME conforme à l'invention pour une application endoscopique,
- la figure 6 illustre un DME conforme à l'invention dont le tube filiforme forme un cathéter,
- la figure 7 illustre schématiquement un DME conforme à l'invention mis en oeuvre dans l'aine de la jambe pour la mesure de PV relatives au coeur et/ou au sang à l'aide d'un cathéter,
- la figure 8, illustre schématiquement la mise en oeuvre d'un DEM dont le tube filiforme forme un laparoscope,
- la figure 9, illustre la mise en oeuvre d'un dispositif conforme à l'invention pour déterminer les PV des tissus appartenant à la cavité abdominale,
- la figure 10 représente schématiquement la mise en oeuvre d'un DEM dont le tube filiforme forme une aiguille,
- la figure 11 illustre une sonde adaptée pour différents type d'aiguille,
- la figure 12 est un graphe relatif à la force exercée par la sonde sur le tissu.

Pour la suite de la description, on entend par élastographie vibratoire une technique de mesure des PV dans laquelle un générateur de vibration génère, par contact direct ou indirect avec un tissu, une ou plusieurs ondes élastiques basse fréquence se propageant dans ce tissu.

La forme temporelle de cette onde élastique basse fréquence peut être arbitraire, mais plus généralement de type impulsionnelle, transitoire ou périodique (entretenue, monochromatique).

Cette vibration est en général obtenue de manière mécanique mais peut également être obtenue par pression de radiation, par hyperthermie ultrasonore ou par des vibrations internes du corps (battements cardiaque, pouls, etc.). De même, la vibration peut être obtenue à l'aide d'un générateur de vibration situé à l'extérieur du corps.

En outre, différents types de transducteurs ultrasonore monoélément ou multiélément peuvent être utilisés dans le DEM de l'invention. Il peut s'agir par exemple, et de manière non limitative, d'un transducteur de type couronne, annulaire, matrice 2D, barrette linéaire ou convexe, d'un transducteur monoélément, d'un transducteur triélément, d'un transducteur de type étoilé, etc...

A titre illustratif et en référence à la figure 2, un dispositif 21 de micro-élastographie, conforme à l'invention est muni d'une sonde 20, composée d'un générateur de vibration 22 générant des ondes élastiques basse fréquence et d'au moins un transducteur ultrasonore 23, laquelle sonde est reliée à un contrôleur 24 maintenu à l'extérieur du corps 27, par un tube filiforme 25 flexible et libre de contraintes angulaires, plus précisément mobile dans l'espace sans aucune contrainte mécanique du fait de sa ductilité, notamment la souplesse et la flexibilité.

La sonde est acheminée par une voie naturelle 26 tel que par exemple le tractus-gastrointestinal, au contact d'un organe 28, appartenant au corps humain ou animal dont ont souhaite déterminer les propriétés viscoélastiques. Cette mise en oeuvre avantageuse permet de venir en contact des organes profonds du corps humain ou animal par des voies naturelles c'est-à-dire de manière non invasive pour déterminer précisément des données qualitatives et/ou quantitatives relatives aux propriétés viscoélastiques.

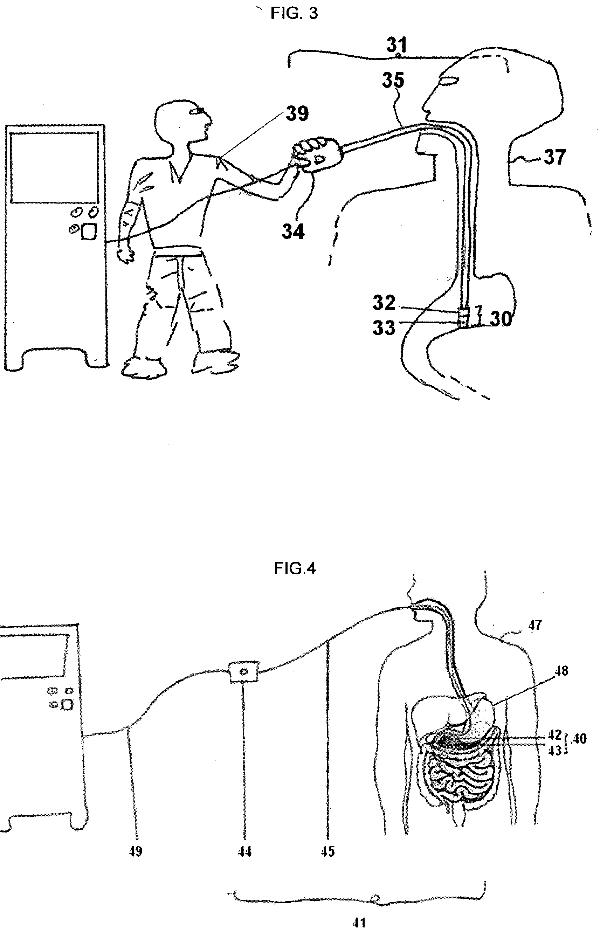

En référence à la figure 3, un dispositif 31 de micro-élastographie, conforme à l'invention est muni d'un générateur de vibration 32 générant des ondes élastiques basse fréquence et d'au moins un transducteur ultrasonore 33 commandés par un contrôleur externe 34.
Le générateur de vibration 32 et le transducteur ultrasonore 33 sont reliés au contrôleur 34 par un tube filiforme 35 flexible, libre de contraintes angulaires, permettant l'introduction du générateur de vibration 32 et d'au moins un transducteur ultrasonore 33 dans le corps humain 37 tandis que le contrôleur 34 est maintenu hors de ce corps.

Ainsi, un utilisateur 39 utilisant le DEM 31 pour acquérir des données peut amener la sonde 30 de ce DEM au voisinage ou au contact de tissus profonds pour effectuer la mesure de leur PV en s'affranchissant des problèmes liés à la présence d'une couche adipeuse à proximité de l'épiderme ou à la diffraction des ondes émises comme décrit précédemment avec l'art antérieur.

Comme représenté sur la figure 4, un être humain présente de nombreuses voies naturelles permettant un tel acheminement de la sonde d'un DME conforme à l'invention dans un organisme, notamment les voies aériennes supérieures - telles que le nez ou la bouche - et les voies inférieures - telles que le rectum - peuvent permettre une introduction d'une sonde d'un DME dans le corps humain.

Par exemple, la sonde 40 d'un DME 41 peut être introduite par la voie aérienne supérieure d'un humain 47 pour être acheminée, grâce au tube filiforme 45 souple et flexible, via le tractus gastro-intestinal, dans l'estomac 48. Lorsque la sonde 40 est au voisinage ou au contact du tissu à analyser, une vibration mécanique est générée par le générateur de vibration de la sonde 40 pour transmettre une ou plusieurs ondes élastiques basse fréquence dans ce tissu à analyser.

Afin de suivre le déplacement de cette ou de ces ondes élastiques basse fréquence, le transducteur ultrasonore de la sonde 40 émet et acquiert simultanément des ondes ultrasonores haute fréquence. Les signaux ultrasonores reçus sont traités afin de mesurer les déplacements engendrés dans ledit milieu par cette ou ces ondes élastiques basse fréquence.

L'évolution spatio-temporelle de ces déplacements permet d'obtenir des données quantitatives et qualitatives relatives aux PV du tissu analysé conformément aux techniques d'élastographie.

La commande du générateur de vibration et du transducteur ultrasonore est effectuée à partir d'un contrôleur 44 situé à l'extérieur du corps du patient 47. Plus précisément, ce contrôleur 44 permet de commander une source d'alimentation transmettant l'énergie nécessaire au fonctionnement du générateur de vibration ou du transducteur ultrasonore. À ce titre, cette énergie est transmise jusqu'au contrôleur 44 via une liaison filaire 49. À partir du contrôleur 44, cette énergie est transmise jusqu'au générateur de vibration et au transducteur ultrasonore via les moyens de liaison mécanique formés par un tube filiforme 45.

Ainsi le DME 41 permet de mesurer les PV de tissus contre lesquels il vient en contact direct mais également les PV de tissus situés à proximité de ces tissus de contact.

Par exemple, ladite sonde 40 d'un DEM 41 est en contact avec la paroi de l'estomac 48 du patient 47. Cette configuration spatiale offre la possibilité de déterminer les PV du tissu de l'estomac 48 mais également les PV du foie étant donné que ces deux organes - foie et estomac - sont proches, au voisinage et en contact, direct ou indirect, avec la sonde 40.

En résumé et d'une façon générale, la sonde est introduite dans un corps, par exemple par une voie naturelle, jusqu'à ce qu'une de ses extrémités soit en contact, direct ou indirect, avec un tissu dont des PV sont à déterminer.

Après cette mise en contact, direct ou indirecte, un procédé d'élastographie vibratoire est utilisé. Une vibration basse fréquence de forme arbitraire, impulsionnelle, transitoire ou périodique est générée en utilisant un générateur d'onde élastique basse fréquence dans les tissus biologiques et en mesurant, par l'intermédiaire d'au moins un transducteur ultrasonore, la réponse du tissu biologique à cette ou ces ondes élastiques basse fréquence.

Les DEM 31, 41 décrits précédemment peuvent être mis en oeuvre sous la forme de cathéters, de dispositifs endoscopiques, les sondes 30, et 40 formant l'extrémité distale du dispositif endoscopique tandis que le contrôleur 34 ou 44 se situe à l'extrémité proximale.

Les DEM 31 ou 41 décrits précédemment peuvent également être mis en oeuvre sous la forme d'accessoires destinés entre autre à être introduits dans le canal opérateur d'un endoscope, les sondes 30, 40 formant l'extrémité distale de l'accessoire tandis que le contrôleur 34, ou 44 se situent à l'extrémité proximal.

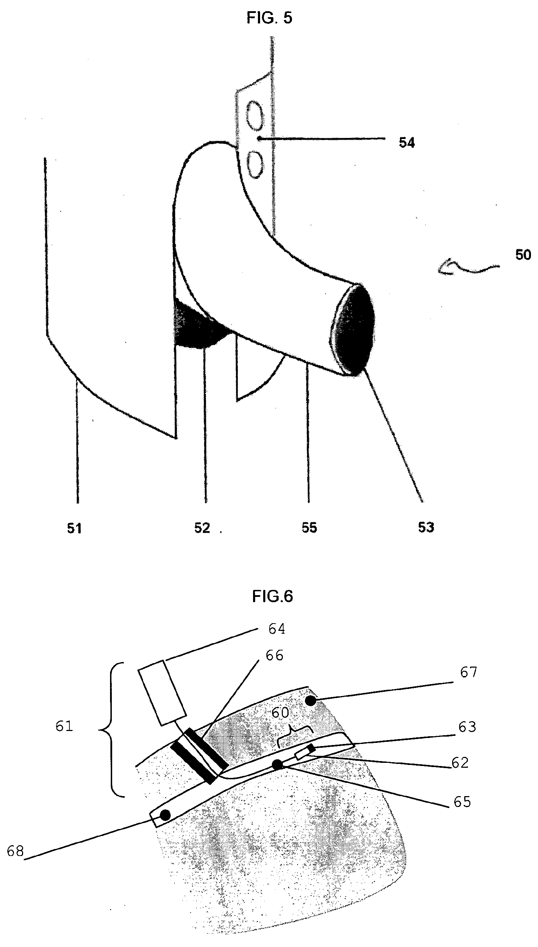

Notamment sur la figure 5 est représentée une mise en oeuvre d'un DEM conforme à l'invention sous la forme d'un accessoire introduit dans le canal opérateur d'un endoscope et dont l'extrémité distale comprend une sonde 50 adaptée à l'endoscopie, laquelle est composée d'un transducteur 53 et d'un générateur de vibration non référencé. Dans cette réalisation, le tube filiforme 55 est introduit à travers le canal opérateur d'un duodénoscope 51 muni d'un érecteur 52 dont le rôle principal est d'orienter et de positionner la sonde 50 en regard des tissus à mesurer. Idéalement l'endoscope 51 comprend également un système de visualisation 54, par exemple optique ou échographique, pour permettre le guidage de l'extrémité distale du DME jusqu'aux tissus dont les PV doivent être mesurées. Ainsi, le système de visualisation annexé au DME facilite l'acheminement mais également son positionnement et offre au praticien la possibilité de s'assurer que le tissu visualisé correspond au tissu souhaité.

Un tel système de visualisation permet de valider la perpendicularité de la sonde avec le tissu à mesurer en vue d'assurer une propagation optimale des ondes élastiques dans les tissus. En effet, une propagation de l'onde basse fréquence dans une direction différente de celle des ultrasons n'est pas favorable à une mesure fiable de sa vitesse et donc des PV du tissu biologique humain ou animal.

Selon une mise en oeuvre différente représentée sur la figure 6 un dispositif de micro-élastographie 61 conforme à l'invention est muni d'une sonde 60 composée d'un générateur d'onde(s) élastique(s) basse fréquence 62 et d'au moins un transducteur ultrasonore 63,laquelle sonde est reliée à un contrôleur 64 maintenu à l'extérieur du corps 67 par un tube filiforme 65 flexible et libre de contrainte angulaire. Selon cette mise en oeuvre ladite sonde 60 est introduite dans un premier temps, au travers d'une voie formée par un instrument 66, comme par exemple un trocart, une canule, autorisant ainsi dans un second temps, l'introduction de ladite sonde 60 au sein d'une voie naturelle 68.

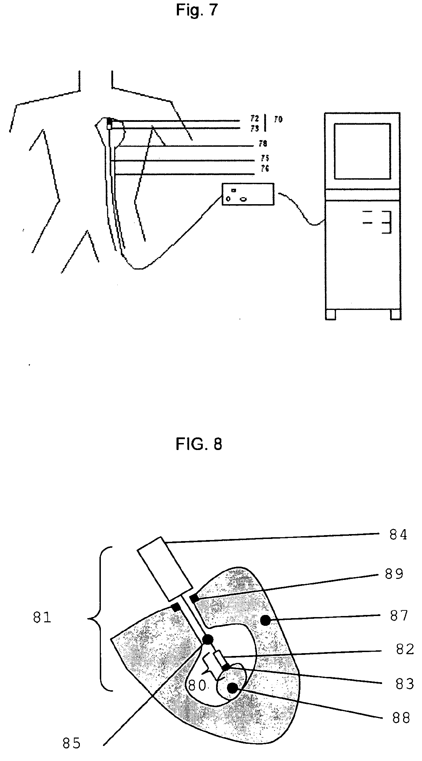

A titre illustratif et en référence à la figure 7 est décrit une réalisation de l'invention mettant en oeuvre un tube filiforme formé par un cathéter 75, ledit tube filiforme servant de moyen de liaison mécanique et de guide présente une souplesse requise pour suivre les vaisseaux sanguins sans pour autant les endommager. En outre, son faible diamètre, typiquement inférieur à 3 mm, est adapté pour être introduit dans les vaisseaux sanguins. Un tel DEM peut être introduit dans une artère, dans une veine, dans un capillaire ou dans tout autre type de vaisseau, par exemple au niveau haut d'une cuisse - l'aine 76- d'un bras, ou d'une jugulaire afin de déterminer les PV des parois dudit réseau sanguin, du sang ou d'un organe tel que le coeur 78.

Par ailleurs, comme représenté sur le figure 8, un dispositif conforme à l'invention 81 de micro-élastographie, est muni d'une sonde 80, composée d'un générateur de vibration 82 générant des ondes élastiques basse fréquence et d'au moins un transducteur ultrasonore 83, laquelle sonde est reliée à un contrôleur 84 maintenu à l'extérieur du corps 87, par un tube filiforme rigide 85. Conformément à cette mise en oeuvre la sonde 80 est acheminée au contact ou au voisinage de l'organe 88 par une voie artificielle 89 formée par un instrument tel que par exemple un écarteur chirurgical, une canule, un trocart ou tout autre type de tige cylindrique creuse autorisant le passage de la sonde et du tube filiforme.

Selon une variante offerte par l'invention le générateur de vibration peut être le tube filiforme 85.

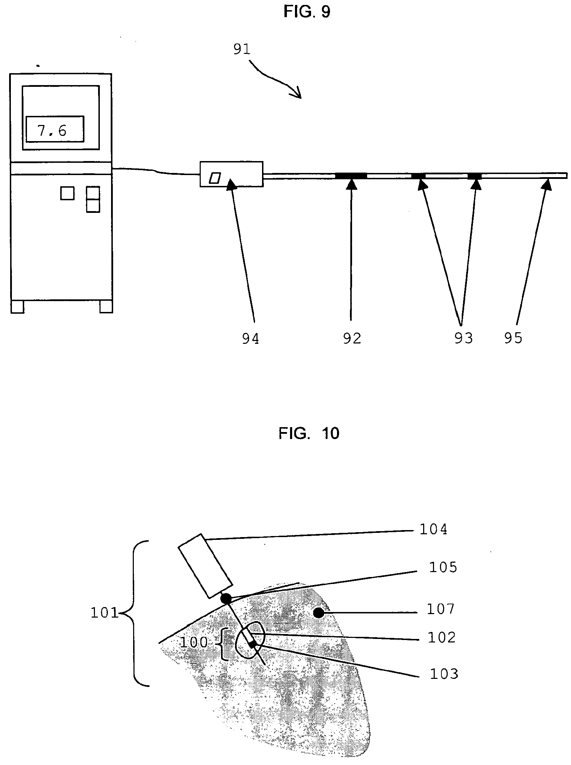

En accord avec la figure 9, le tube filiforme 95 est formé par un endoscope de type flexible ou rigide tel qu'un laparoscope et permet ainsi de visualiser les organes et les tissus de l'abdomen.

En outre, le ou les transducteur(s) ultrasonore 93 ainsi que le générateur de vibration 92 peuvent être positionné à l'extrémité distale du DEM ou tout autre endroit appartenant au tube filiforme tel que représenter sur le schéma.

La figure 10 illustre une mise en oeuvre différente dans laquelle un dispositif conforme à l'invention 101 de micro-élastographie, est muni d'une sonde 100, composée d'un générateur de vibration 102 générant des ondes élastiques basse fréquence et d'au moins un transducteur ultrasonore 103, laquelle sonde est reliée à un contrôleur 104 maintenu à l'extérieur du corps 107, par un tube filiforme rigide ou souple de type aiguille 105. Ladite aiguille acheminée par voie percutanée peut être une aiguille de type biopsie permettant ainsi au praticien de déterminer la valeur des PV des tissus avoisinants l'aiguille au fur et à mesure de son insertion pour guider le praticien lors de l'acheminement de la sonde au site de ponction et permet au praticien de s'assurer que l'échantillon de tissu que l'on souhaite biopser correspond à un tissu dont les PV ont été modifiées.

Selon une variante de l'invention non illustrée le tube filiforme formé par l'aiguille est le générateur de vibration, cette vibration est transmise par l'aiguille.

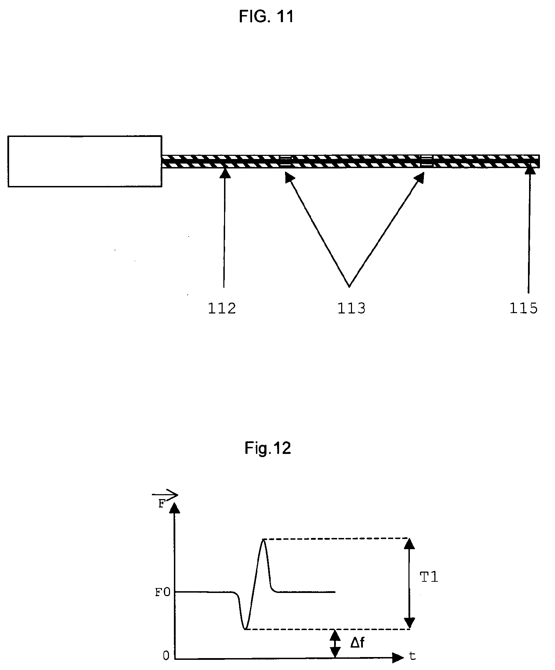

Selon une variante de l'invention supplémentaire illustrée figure 11, le tube filiforme est un accessoire tel qu'un fourreau 112 comprenant au moins un transducteur ultrasonore 113, lequel fourreau recouvre une aiguille 115.

En outre, de manière générale et non limitatives des accessoires peuvent également être adjoints à la sonde d'un DME tel que décrits dans les différentes mises en oeuvre comme des pinces, un ballon gonflable, une lame coupante, une fibre optique, une caméra vidéo ou un système échographique, etc.

De plus, selon une possibilité offerte par l'invention un DME conforme à l'invention peut être utilisé pour déterminer les effets d'un traitement par génération d'ultrasons focalisés de haute intensité « HIFU ». À ce titre, cette mise en oeuvre permet au praticien de connaître à tout moment si le tissu pathologique à été détruit et de décider d'un éventuel arrêt du traitement.

Comme précédemment indiqué, une source d'alimentation externe au DEM transmet l'énergie nécessaire à la génération de ou des ondes élastiques basse fréquence et des ondes ultrasonores via une liaison filaire puis via des moyens de liaison mécanique.

Selon différentes variantes de l'invention la transmission de l'onde basse fréquence ou des ondes basse fréquence s'effectue par un système utilisant l'énergie pneumatique, de l'énergie hydraulique ou de l'énergie électrique activant un micro-mécanisme situé dans la sonde.

Avantageusement le dispositif est muni d'un dispositif d'asservissement en position de l'extrémité du DME.

Dans le cadre de la mesure des PV des tissus, une ou des ondes élastiques basse fréquence comprise entre 5 Hz et 2000 Hz et plus généralement entre 10 Hz et 1000 Hz est engendrée. Afin de suivre le déplacement de l'onde élastique ou des ondes élastiques basse fréquence et ainsi en déduire la vitesse de déplacement, une série d'émissions d'ondes ultrasonores est alors effectuée simultanément à l'émission de ou des ondes élastiques basse fréquence. Les tirs ultrasonores sont effectués avec un intervalle de temps variant entre 1µs et 100ms et plus généralement entre 100µs et 1ms. Les émissions ultrasonores sont associées à des réceptions des signaux ultrasonores constitués par la superposition des échos réfléchis par les diffuseurs présents dans le milieu étudié.

Ces tirs ultrasonores sont généralement effectués sur une durée minimum correspondant à la période de l'onde basse fréquence (inverse de la fréquence) soit 0.5 ms pour une fréquence de 2000 Hz et peuvent être réalisés de manière permanente pendant toute la durée de l'examen. La durée minimum de 1 ms correspond au temps minimum nécessaire pour observer la propagation d'une ou des ondes élastiques basses fréquence afin de déterminer les propriétés viscoélastiques du tissu.

Le nombre et la cadence des tirs ultrasonores dépendent donc de la fréquence des ondes élastiques basse fréquence et de la profondeur mesurée.

Afin d'engendrer une onde élastiques basse fréquence, il est souhaitable que la sonde reste en contact avec les tissus. À titre de comparaison, il convient de rappeler qu'une mesure élastographique selon l'art antérieur requiert une force de l'ordre de 4N à 8N entre le générateur et la peau afin d'assurer la propagation de l'onde jusqu'aux tissus à mesurer. Afin de maintenir ce contact en dehors de l'intervention de l'utilisateur du DEM, une précontrainte peut être mise en oeuvre afin de générer une force statique F0 comme montré sur la figure 12 où l'axe des ordonnées représente la force exercée tandis que l'axe des abscisses représente le temps.

Par la suite, une impulsion T1 peut être donnée de telle manière qu'une variation de force soit positive (Δf > 0) et engendre l'onde basse fréquence tout en maintenant le transducteur ultrasonore en contact avec les tissus.

Selon une possibilité offerte par l'invention le maintien de la force statique F0 et la génération du coup basse fréquence peuvent être effectués à l'aide d'un des éléments suivants : un ressort, un élastomère, un appareil pneumatique, un appareil hydraulique ou musculaire ou tout type d'élément ayant pour caractéristique le maintien de la force statique pendant toute la durée de la période d'acquisition.

Selon une variante non illustrée, l'onde basse fréquence est engendrée différemment par un second dispositif inséré au sein du tube filiforme. Ce tube filiforme pouvant être le canal opérateur d'un endoscope.

L'onde élastique ou les ondes élastiques basse fréquence peuvent également être engendrées par un transducteur ultrasonore externe en mode pression de radiation ou un vibreur situé à l'extérieur du corps.

Selon une variante supplémentaire non illustrée, les ondes élastiques considérées sont issus de déplacements d'organes situés dans le corps, tels que les battements cardiaques.

Une protection, transparente aux ultrasons et dont ses caractéristiques d'élasticité sont proches de celles des tissus dont on souhaite déterminer les PV, peut être située sur la sonde afin de protéger cette dernière des effets corrosifs et/ou de respecter les conditions de biocompatibilité et de stérilité requis et de diminuer les risques de contamination.

Le tube filiforme peut être à titre non limitatifs un cathéter, un tube, un tuyau, un conduit, un canal, une gaine, un endoscope, une aiguille mais également tout autre moyen de liaison souple, flexible, rigide possédant une longueur supérieur à 20 mm typiquement comprise entre 20 mm et 3 mètres permettant d'acheminer et de positionner la sonde en contact direct ou au voisinage du tissu dont on souhaite déterminer les PV.

Tandis que la sonde est destinée à être introduite dans un corps, le contrôleur externe et la source d'énergie, à laquelle il est relié par une liaison filaire, sont maintenus en dehors du corps considéré afin de permettre à un praticien de commander le transducteur et le générateur de vibration de la sonde.

L'invention permet de s'affranchir des couches graisseuses situées à proximité de l'épiderme de telle sorte que les mesures de PV et leurs interprétations sont simplifiées et améliorées

Par ailleurs, il est connu d'effectuer des mesures de propriétés viscoélastiques sur une profondeur comprise entre 25 et 65 mm, ce qui permet de prendre en compte la présence d'une couche adipeuse.

Dans le cadre de l'invention, le DME est en contact direct avec des tissus dont les épaisseurs sont parfois inférieures à 25 mm, typiquement comprise entre 2 et 12 mm.

Pour permettre la mesure des PV dans ces tissus proches de la sonde, la présente invention génère des ondes élastiques basse fréquence dont la fréquence est comprise entre 10 Hz et 1000 Hz, c'est-à-dire à une fréquence plus élevée que la fréquence classiquement mise en oeuvre pour des mesures de propriété viscoélastique au moyen d'une sonde extérieure au corps humain - de l'ordre de 50 Hz.

De plus, la faible épaisseur des tissus à analyser selon l'invention soulève également un problème de résolution. À ce titre la présente invention se propose d'augmenter la fréquence des ondes ultrasonores utilisées pour suivre les ondes élastiques basse fréquence. Cette fréquence est comprise entre 1 MHz et 200 MHż, et plus spécifiquement entre 5 MHz et 50 MHz, de façon à obtenir une résolution augmentée par rapport à l'art antérieur.

Avantageusement le DME selon l'invention est commandé par au moins un ordinateur, un micro-ordinateur, une unité centrale ou tout type de système de commande, permettant ainsi d'adapter la fréquence de l'onde élastiques ou des ondes élastiques basse fréquence engendrées aux profondeurs de tissus que l'on souhaite visualiser. Grâce à cette particularité, la présente invention propose un DME qui permet d'obtenir une vibration, ou sollicitation, basse fréquence parfaitement contrôlée en temps et en amplitude et adapté à l'épaisseur à mesurer.

La présente invention est décrite dans ce qui précède à titre d'exemple. Il est entendu que l'homme du métier est à même de réaliser différentes variantes de l'invention sans pour autant sortir du cadre du brevet. Ainsi, différents procédés permettent d'obtenir des mesures qualitatives de PV de tissus à l'aide d'un traitement statistique d'une pluralité de mesures. Par exemple, des valeurs présentant un écart type prédéterminé par rapport à la valeur médiane de ces mesures peuvent être écartées.

## Revendications

1. Dispositif d'élastographie vibratoire (21, 31, 61) pour la mesure quantitative et/ou qualitative de propriétés viscoélastiques d'un tissu humain ou animal, ce dispositif (21, 31, 61) étant muni :
- d'une sonde (20, 30, 60) comprenant au moins un transducteur ultrasonore (23, 33, 63) et un générateur de vibration basse fréquence (22, 32, 62), le(s) transducteur(s) ultrasonore (23, 33, 63) générant des ondes ultrasonores permettant l'analyse de la propagation des ondes élastiques basse fréquence se propageant dans un organe profond et générées par le générateur de vibration basse fréquence (22, 32, 62), la sonde (20, 30, 60) étant destinée à être positionnée au voisinage ou au contact de l'organe profond,
- d'un contrôleur (24, 34, 64), relié à la sonde (20, 30 60), comprenant des moyens d'actionnement de la sonde, le contrôleur (24, 34, 64) étant destiné à être maintenu à l'extérieur du corps humain ou animal,
- d'une liaison filaire (49) adaptée pour transmettre de l'énergie audit contrôleur,
- ledit dispositif d'élastographie vibratoire (21, 31, 61) étant **caractérisé en ce qu'**il comporte des moyens de liaison mécanique (25, 35, 65) de la sonde (20, 30, 60) avec le contrôleur (24, 34, 64) pour acheminer et positionner la sonde en contact direct ou au voisinage du tissu de l'organe dont on souhaite déterminer les propriétés viscoélastiques, les moyens de liaison mécanique (25, 35, 65) étant formés par un tube filiforme qui forme un cathéter dont l'extrémité distale contient la sonde (20, 30, 60) et l'extrémité proximale comprend le contrôleur (24, 34, 64).

2. Dispositif (21, 31, 61) selon la revendication 1, **caractérisé en ce que** les moyens de liaison mécanique présentent une longueur supérieure à 20 mm, de préférence comprise entre 20 mm et 3 mètres.

3. Dispositif (21, 31, 61) selon l'une des revendications 1 ou 2, **caractérisé en ce que** le transducteur ultrasonore (23, 33, 63) présente un diamètre actif inférieur à 3 mm.

4. Dispositif (21, 31, 61) selon l'une quelconque des revendications 1 à 3, **caractérisé en ce que** le contrôleur (24, 34, 64) comprend des moyens pour commander la transmission d'énergie au générateur de vibration et/ou au(x) transducteur(s) ultrasonore.

## Patentansprüche

1. Vibrierende Elastographievorrichtung (21, 31, 61) für die quantitative und / oder qualitative Messung von viskoelastischen Eigenschaften eines menschlichen oder tierischen Gewebes, wobei diese Vorrichtung (21, 31, 61) versehen ist mit:
- einer Sonde (20, 30, 60), umfassend wenigstens einen Ultraschalltransduktor (23, 33, 63) und einen Niedrigfrequenz-Vibrationsgenerator (2, 32, 62), wobei der (die) Ultraschalltransduktor(en) (23, 33, 63) Ultraschallwellen generiert / generieren, die die Analyse der Verbreitung der elastischen Niedrigfrequenzwellen zulässt, die sich in einem tiefen Organ verbreiten und durch den Niedrigfrequenzgenerator (22, 32, 62) generiert werden, wobei die Sonde (20, 30, 60) dazu bestimmt ist, in der Nähe oder in Kontakt mit dem tiefen Organ positioniert zu werden,
- eine Kontrollvorrichtung (24, 34, 64), die an die Sonde (20, 30, 60) angeschlossen ist, umfassend Betätigungsmittel der Sonde, wobei die Kontrollvorrichtung (24, 34, 64) dazu bestimmt ist, an der Außenseite des menschlichen oder tierischen Körpers festgehalten zu werden,
- eine drahtgebundene Verbindung (49), die geeignet ist, um die Energie auf die genannte Kontrollvorrichtung zu übertragen,
- wobei die genannte vibrierende Elastographievorrichtung (21, 31, 61) **dadurch gekennzeichnet ist, dass** sie mechanische Verbindungsmittel (25, 35, 65) der Sonde (20, 30, 60)mit der Kontrollvorrichtung (24, 34, 64) zum Verbringen und Positionieren der Sonde in direkten Kontakt mit oder in der Nähe des Gewebes des Organs umfasst, dessen viskoelastische Eigenschaften bestimmt werden sollen, wobei die mechanischen Verbindungsmittel (25, 35, 65) durch eine filiforme Röhre gebildet sind, die einen Katheter bildet, dessen distales Ende die Sonde (20, 30, 60) enthält und das proximale Ende die Kontrollvorrichtung (24, 34, 64) umfasst.

2. Vorrichtung (21, 31, 61) gemäß Anspruch 1, **dadurch gekennzeichnet, dass** die mechanischen Verbindungsmittel eine größere Länge als 20 mm, die bevorzugt zwischen 20 mm und 3 Metern inbegriffen ist, aufweisen.

3. Vorrichtung (21, 31, 61) gemäß Anspruch 1 oder 2, **dadurch gekennzeichnet, dass** der Ultraschalltransduktor (23, 33, 63) einen aktiven Durchmesser von weniger als 3 mm aufweist.

4. Vorrichtung (21, 31, 61) gemäß Anspruch 1 bis 3, **dadurch gekennzeichnet, dass** die Kontrollvorrichtung (24, 34, 64) Mittel umfasst, um die Energieübertragung auf den Vibrationsgenerator und / oder den (die) Ultraschalltransduktor(en) zu steuern.

## Claims

1. Vibratory elastography device (21, 31, 61) for the quantitative and/or qualitative measurement of viscoelastic properties of human or animal tissue, said device (21, 31, 61) being provided with:
- a probe (20, 30, 60) including at least one ultrasonic transducer (23, 33, 63) and a low frequency vibration generator (22, 32, 62), the ultrasonic transducer(s) (23, 33, 63) generating ultrasonic waves enabling the analysis of the propagation of low frequency elastic waves propagating in a deep organ and generated by the low frequency vibration generator (22, 32, 62), the probe (20, 30, 60) being intended to be positioned in the vicinity of or in contact with the deep organ,
- a controller (24, 34, 64), connected to the probe (20, 30, 60), including means of actuating the probe, the controller (24, 34, 64) being intended to be maintained outside of the human or animal body,
- a wire link (49) suited to transmit energy to said controller,
- said vibratory elastography device (21, 31, 61) being **characterised in that** it comprises means of mechanical connection (25, 35, 65) of the probe (20, 30, 60) with the controller (24, 34, 64) to guide and position the probe in direct contact with or in the vicinity of the tissue of the organ of which it is wished to determine the viscoelastic properties, the means of mechanical connection (25, 35, 65) being formed by a filiform tube which forms a catheter of which the distal end contains the probe (20, 30, 60) and the proximal end includes the controller (24, 34, 64).

2. Device (21, 31, 61) according to claim 1, **characterised in that** the means of mechanical connection have a length greater than 20 mm, preferably comprised between 20 mm and 3 metres.

3. Device (21, 31, 61) according to one of claims 1 or 2, **characterised in that** the ultrasonic transducer (23, 33, 63) has an active diameter less than 3 mm.

4. Device (21, 31, 61) according to any of claims 1 to 3, **characterised in that** the controller (24, 34, 64) includes means of commanding the transmission of energy to the vibration generator and/or to the ultrasonic transducer(s).
